# EUROPÄISCHE PATENTSCHRIFT

(11) **EP 4 215 152 B1**
(45) Veröffentlichungstag und Bekanntmachung des Hinweises auf die Patenterteilung: **25.12.2024**
(21) Anmeldenummer: 22152354.1
(22) Anmeldetag: 20.01.2022
(51) Int. Cl.: A61C 13/15, A61B 5/00

(54) **DENTALHANDGERÄT ZUM AUSHÄRTEN VON MATERIAL UND ANREGEN VON FLUORESZENZ**
DENTAL HANDHELD TOOL FOR CURING MATERIAL AND EXCITATION OF FLUORESCENCE
APPAREIL DENTAIRE PORTATIF PERMETTANT DE DURCIR LA MATIÈRE ET D'EXCITER LA FLUORESCENCE

(43) Veröffentlichungstag der Anmeldung: 26.07.2023
(73) Patentinhaber: Ivoclar Vivadent AG, 9494 Schaan (LI)
(72) Erfinder: GLEBOVA, Tatiana, 9470 Buchs (CH); GROSSE-HONEBRINK, Alexander, 9463 Oberriet (CH); SENN, Bruno, 9056 Gais (CH)
(74) Vertreter: Baldus, Oliver

(56) Entgegenhaltungen:
- WO-A1-2011/035372
- US-B1- 6 325 623

## Beschreibung

Die vorliegende Erfindung betrifft ein Dentalhandgerät zum Aushärten einer lichthärtenden Substanz mit Licht in einem ersten Wellenlängenbereich und zum Anregen einer Fluoreszenz von Zahnsubstanz mit Licht in einem zweiten Wellenlängenbereich. Zahnärztliche Diagnosen werden heute unter Verwendung der zahnärztlichen Expertise bei der visuellen Begutachtung des Zahnstatus sowie mittels Röntgenaufnahmen gestellt. Neben Röntgenaufnahmen gibt es heute Intraoralscanner und Intraoralkameras, die langwellige (Infrarot)-Strahlung zur Transillumination der Zähne nutzen. Mit dieser Methode lässt sich Approximalkaries darstellen. Ein Zahnarzt kann optisch gewisse Indikationen erkennen. Es ist jedoch oft schwierig Restaurationsmaterialien und Zahnsubstanz von Auge auseinander zu halten. Für dieses Problem gibt es zurzeit keine einfache Lösung.

WO 2011/035372 A1 offenbart ein Gerät, das alle technischen Merkmalen der Präambel von Anspruch 1 aufweist.

Es ist die Aufgabe der vorliegenden Erfindung, ein Dentalhandgerät zum Aushärten einer lichthärtenden Substanz bereitzustellen, mit dem zusätzlich bestimmte Stellen in einem Zahngebiss sichtbar gemacht werden können.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird diese technische Aufgabe durch ein Dentalhandgerät gelöst, mit einer Aushärtevorrichtung zum Aushärten eines Zahnrestaurationsmaterials mit Licht in einem ersten Wellenlängenbereich; und einer Fluoreszenzanregungsvorrichtung zum Anregen einer Fluoreszenz der Zahnsubstanz mit Licht in einem zweiten Wellenlängenbereich; und einem elektronischen Steuergerät zum Steuern der Aushärtevorrichtung und der Fluoreszenzanregungsvorrichtung; wobei, eine Intensität des Lichtes der Aushärtevorrichtung und/oder des Lichtes der Fluoreszenzanregungsvorrichtung einstellbar ist.

Das Dentalhandgerät erlaubt einem Benutzer Restaurationen und Karies in einer Kavität auf einfache Weise zu erkennen und von gesunder Zahnsubstanz zu unterscheiden. Zusätzlich kann die Anregung von bakteriellen Metaboliten zur Identifikation und Bestätigung von anderen Diagnosen (Plaque, Zahnstein) eingesetzt werden. Der Vorteil der Kombination von Aushärtevorrichtung, wie beispielsweise einer Polymerisationslampe, und Fluoreszenzvorrichtung ist, dass während, vor oder nach einer Aushärtung von Zahnrestaurationsmaterial auch eine Identifikation von kariösen Stellen durchgeführt werden kann.

Durch das Steuergerät wird beispielsweise der technische Vorteil erreicht, dass die Aushärtevorrichtung und die Fluoreszenzanregungsvorrichtung flexibel und unabhängig gesteuert werden können.

In einer weiteren technisch vorteilhaften Ausführungsform des Dentalhandgeräts ist das elektronische Steuergerät ausgebildet, die Aushärtevorrichtung und die Fluoreszenzanregungsvorrichtung jeweils unabhängig voneinander zu aktivieren. Dadurch wird beispielsweise der technische Vorteil erreicht, dass die Aushärtevorrichtung und die Fluoreszenzanregungsvorrichtung nach Bedarf ein- und ausgeschaltet werden können.

In einer weiteren technisch vorteilhaften Ausführungsform des Dentalhandgeräts ist das elektronische Steuergerät ausgebildet, die Aushärtevorrichtung und die Fluoreszenzanregungsvorrichtung in einem vorgegebenen zeitlichen Abstand zu aktivieren. Dadurch wird beispielsweise der technische Vorteil erreicht, dass automatisch nach einer Aushärtung die Überprüfung einer ausgehärteten Füllung durchgeführt werden kann.

In einer weiteren technisch vorteilhaften Ausführungsform des Dentalhandgeräts umfasst das Dentalhandgerät eine elektronische Kamera zum Aufnehmen von Fluoreszenzlicht. Vor der Kamera kann ein optisches Filter angeordnet sein, um beispielsweise Blaulicht herauszufiltern. Durch die Kamera wird beispielsweise der technische Vorteil erreicht, dass Fluoreszenzbilder kariöser Stellen abgespeichert werden können.

In einer weiteren technisch vorteilhaften Ausführungsform des Dentalhandgeräts umfasst das Dentalhandgerät eine elektronische Schnittstelle zum Übertragen von Daten der elektronischen Kamera an ein externes Gerät. Dadurch wird beispielsweise der technische Vorteil erreicht, dass digitale Fluoreszenzbilder kariöser Stellen extern verarbeitet und ausgewertet werden können.

In einer weiteren technisch vorteilhaften Ausführungsform des Dentalhandgeräts umfasst das Dentalhandgerät eine einzige Lichtquelle, die ausgebildet ist, Licht in dem ersten Wellenlängenbereich und dem zweiten Wellenlängenbereich zu emittieren. Dadurch wird beispielsweise der technische Vorteil erreicht, dass das Dentalhandgerät mit geringem Aufwand hergestellt werden kann.

In einer weiteren technisch vorteilhaften Ausführungsform des Dentalhandgeräts umfasst das Dentalhandgerät ein optisches Filter zum Sperren des ersten Wellenlängenbereichs oder des zweiten Wellenlängenbereichs. Dadurch wird beispielsweise der technische Vorteil erreicht, dass sich der Wellenlängenbereich des jeweiligen Lichtes mit hoher Genauigkeit einstellen lässt.

In einer weiteren technisch vorteilhaften Ausführungsform des Dentalhandgeräts ist ein Strahlengang durch das optische Filter umlenkbar oder das Filter in den Strahlengang bewegbar. Dadurch wird beispielsweise der technische Vorteil erreicht, dass sich die Frequenz des jeweiligen Lichts steuern lässt.

In einer weiteren technisch vorteilhaften Ausführungsform des Dentalhandgeräts umfasst das Dentalhandgerät einen beweglichen Spiegel oder einen elektronisch ansteuerbarer Mikrospiegel zum Umlenken des Strahlengangs durch das optische Filter. Dadurch wird beispielsweise der technische Vorteil erreicht, dass der Strahlengang auf einfache Weise umgelenkt werden kann.

In einer weiteren technisch vorteilhaften Ausführungsform des Dentalhandgeräts umfasst das Filter ein Kantenfilter oder ein Hochpassfilter. Dadurch wird beispielsweise der technische Vorteil erreicht, dass ein nicht verwendeter Wellenlängenbereich wirksam unterdrückt werden kann.

In einer weiteren technisch vorteilhaften Ausführungsform des Dentalhandgeräts umfasst das Dentalhandgerät eine erste Lichtquelle zum Emittieren von Licht in dem ersten Wellenlängenbereich und eine zweite Lichtquelle zum Emittieren von Licht in dem zweiten Wellenlängenbereich. Dadurch wird beispielsweise der technische Vorteil erreicht, dass für jeden Anwendungszweck eine spezielle Lichtquelle verwendet werden kann, die unabhängig von der anderen Lichtquelle ist und Energie gespart werden kann.

Gemäß der Erfindung des Dentalhandgeräts ist eine Intensität des Lichtes der Aushärtevorrichtung und/oder des Lichtes der Fluoreszenzanregungsvorrichtung einstellbar. Zu diesem Zweck ist das Steuergerät mit dem die Intensität des Lichtes der Aushärtevorrichtung und/oder des Lichtes der Fluoreszenzanregungsvorrichtung unabhängig voneinander einstellbar ist. Das Steuergerät kann beispielsweise die elektrische Leistung der jeweiligen Lichtquelle steuern. Dadurch wird beispielsweise der technische Vorteil erreicht, dass das Dentalhandgerät an spezielle Lichtverhältnisse angepasst werden kann.

In einer weiteren technisch vorteilhaften Ausführungsform des Dentalhandgeräts ist eine Aktivierung der Aushärtevorrichtung und/oder der Fluoreszenzanregungsvorrichtung auswählbar. Zu diesem Zweck kann in dem Dentalhandgerät ein elektronischer Schalter angeordnet sein, durch den die Aktivierung der Aushärtevorrichtung und/oder der Fluoreszenzanregungsvorrichtung manuell auswählbar ist. Dadurch wird beispielsweise der technische Vorteil erreicht, dass ein Benutzer je nach Bedarf eine Fluoreszenz anregen kann oder das Zahnrestaurationsmaterial aushärten kann.

In einer weiteren technisch vorteilhaften Ausführungsform des Dentalhandgeräts liegt der erste Wellenlängenbereich zwischen 380 nm und 515 nm und der zweite Wellenlängenbereich zwischen 380 nm und 415 nm. Dadurch wird beispielsweise der technische Vorteil erreicht, dass sich das Zahnrestaurationsmaterial effizient aushärten lässt und kariöse Stellen gut erkennen lassen. Durch den zweiten Wellenlängenbereich kann ein Überstrahlen der Fluoreszenz verhindert werden.

Ausführungsbeispiele der Erfindung sind in den Zeichnungen dargestellt und werden im Folgenden näher beschrieben.

Es zeigen:
- Fig. 1: eine schematische Ansicht eines Dentalhandgeräts;
- Fig. 2: ein Spektrum einer Lichtquelle des Dentalhandgerätes; und
- Fig. 3: ein weiteres Spektrum einer Lichtquelle des Dentalhandgerätes.

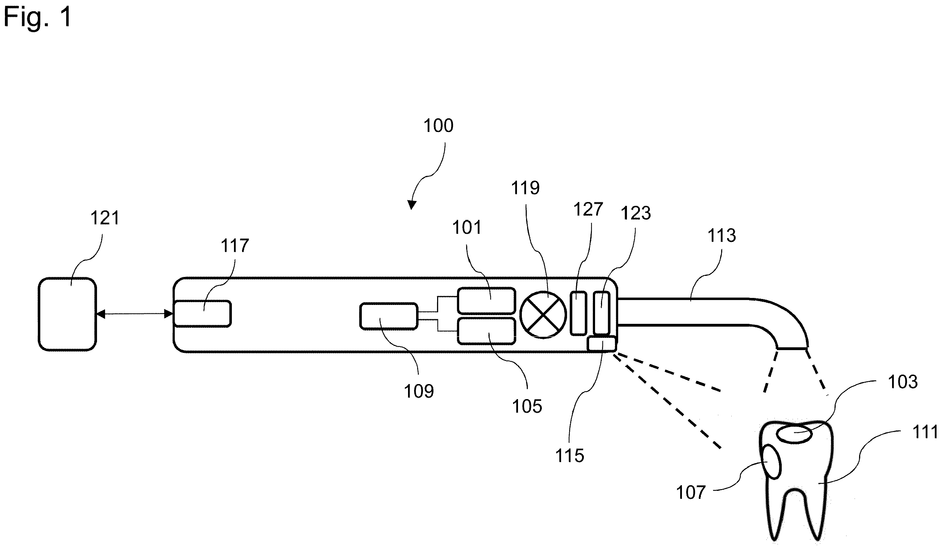

Fig. 1 zeigt eine schematische Ansicht eines Dentalhandgeräts 100. Das Dentalhandgerät 100 ist ein tragbares elektronisches Gerät, das von einem Benutzer während der Verwendung in der Hand gehalten werden kann. Das Dentalhandgerät 100 umfasst eine

Aushärtevorrichtung 101 zum Aushärten eines Zahnrestaurationsmaterials 103 mit Licht in einem ersten Wellenlängenbereich. Das Licht wird von einer Lichtquelle 119 innerhalb des Dentalhandgerätes 100 emittiert. Die Lichtquelle 119 ist beispielsweise durch eine Leuchtdiode oder eine Laserdiode gebildet. Das Licht wird mittels eines Lichtleiters 113 zu einem Zahn 111 geführt, in dem ein Zahnrestaurationsmaterial 103 angeordnet ist. Das Zahnrestaurationsmaterial 103 ist beispielsweise ein Polymer, das zwischen 380 - 515 nm photoinduziert aushärtbar ist.

Zusätzlich zur Aushärtevorrichtung 101 umfasst das Dentalhandgerät 100 eine Fluoreszenzanregungsvorrichtung 105 zum Anregen einer Fluoreszenz von kariöser Zahnsubstanz 107 eines Zahns 111 mit Licht in einem ersten Wellenlängenbereich. Durch die Fluoreszenzanregungsvorrichtung 105 ist es beispielsweise möglich, eine Fluoreszenz von bakteriellen Metaboliten mit blauem Licht anzuregen. Eine stattfindende Fluoreszenz ist ein Indikator für die Gegenwart oraler Mikroorganismen und ein Indiz für orale Zustände, wie beispielsweise Plaque, Zahnstein und Karies. Fluoreszenz ist die spontane Emission von Licht kurz nach der Anregung eines Materials durch Licht. Dabei sind die emittierten Photonen in der Regel energieärmer als die vorher absorbierten. Das Aushärten des Zahnrestaurationsmaterials 103 kann in beiden Wellenlängenbereichen geschehen, während die Fluoreszenzanregung nur im zweiten Wellenlängenbereich stattfindet.

Die Aushärtevorrichtung 101 und die Fluoreszenzanregungsvorrichtung 105 werden durch ein elektronisches Steuergerät 109 gesteuert. Das Steuergerät 109 ist ausgebildet, die Aushärtevorrichtung 101 und die Fluoreszenzanregungsvorrichtung 105 jeweils unabhängig voneinander zu aktivieren oder in einem vorgegebenen zeitlichen Abstand zu aktivieren, wie beispielsweise 3 Sekunden. Dadurch kann beispielsweise die Fluoreszenzanregungsvorrichtung 105 nach einer vorgegebenen Zeitspanne nach dem Deaktivieren der Aushärtevorrichtung 101 aktiviert werden. Dadurch kann sichergestellt werden, dass nach einer Aushärtung des Zahnrestaurationsmaterials 103 an einer kariösen Stelle stets überprüft wird, ob weitere kariöse Stellen vorhanden sind. Das Steuergerät 109 kann beispielsweise einen Mikroprozessor und einen digitalen Speicher umfassen, in dem Steuerprogramme und Daten abgelegt sind.

Zudem kann das Dentalhandgerät 100 eine elektronische Kamera 115 umfassen, mit der eine digitale Aufnahme des vom Zahn 111 erzeugten Fluoreszenzlichtes erhalten werden kann, wie beispielsweise eine CCD-Kamera. Die elektronische Kamera 115 kann ebenfalls durch das elektronische Steuergerät 109 aktiviert werden. Durch die digitale Aufnahme kann eine auftretende Fluoreszenz dauerhaft als Bilddatei dokumentiert werden. Über eine elektronische Datenschnittstelle 117 kann die Bilddatei dann an ein externes Gerät 121 übermittelt werden. Die Datenschnittstelle ist beispielsweise durch eine WLAN- oder Bluetooth-Schnittstelle gebildet. An dem externen Gerät 121, wie beispielsweise einem Computer, kann die digitale Aufnahme analysiert und in eine Krankenakte exportiert werden.

Das Dentalhandgerät 100 kann eine breitbandige Lichtquelle 119 umfassen, die sowohl Licht mit dem ersten Wellenlängenbereich als auch dem zweiten Wellenlängenbereich ausgibt. Das Dentalhandgerät 100 kann aber auch zwei getrennte schmalbandige Lichtquellen 119 umfassen, von denen eine den ersten Wellenlängenbereich ausgibt und der Aushärtevorrichtung 101 zugeordnet ist und eine andere den zweiten Wellenlängenbereich ausgibt und der Fluoreszenzanregungsvorrichtung 105 zugeordnet ist. Diese Lichtquellen lassen sich unabhängig voneinander ein- und ausschalten.

Beim Aushärten können aber auch zwei Lichtquellen 119 mit unterschiedlichen Wellenlängenbereichen gleichzeitig eingeschaltet sein, wie beispielsweise eine Leuchtdiode mit einer Wellenlänge von 410 nm und eine Leuchtdiode mit einer Wellenlänge von 465 nm, während bei einer Fluoreszenzanregung nur eine einzige Lichtquelle eingeschaltet ist, wie beispielsweise die Leuchtdiode mit einer Wellenlänge von 410 nm. Somit sind mehrere Lichtquellen 119 in der Aushärtewellenlänge eingeschaltet. Zusätzlich können mehrere Leuchtmittel als Lichtquelle 119 für einen Wellenlängenbereich eingeschaltet sein, wie beispielsweise mehrere Leuchtdioden mit einer Wellenlänge von 465 nm, um eine Leistung zu steigern.

Wird nur eine einzige Lichtquelle 119 verwendet, umfasst das Dentalhandgerät 100 ein optisches Filter 123 zum Sperren des ersten Wellenlängenbereichs und/oder des zweiten Wellenlängenbereich. Das optische Filter 123 ist beispielsweise ein Bandpassfilter oder ein Kantenfilter. Je nach verwendetem Wellenlängenbereich kann der Strahlengang des Lichtes durch das optische Filter 123 gelenkt oder das optische Filter 123 in den Strahlengang bewegt werden. Die Steuerung hierzu wird von dem elektronisches Steuergerät 109 durchgeführt.

Dies kann durch einen mechanisch beweglichen Spiegel 127 oder ein Feld elektronisch ansteuerbarer Mikrospiegel (DLP - Digital Light Processing) zum Umlenken des Strahlengangs durch das optische Filter 123 erreicht werden. Durch das Feld elektronisch ansteuerbarer Mikrospiegel wird der Lichtstrahl durch eine rechteckige Anordnung von beweglichen Mikrospiegeln in Pixel zerlegt und dann pixelweise entweder in den Projektionsweg hinein oder aus dem Projektionsweg hinaus reflektiert.

Zudem kann das blaue Licht der Fluoreszenzanregungsvorrichtung 105 eine grünliche Fluoreszenz von zahnärztlichen Materialien anregen. In einigen zahnärztlichen Materialien, die zur Füllung von kariösen Läsionen verwendet werden, sind fluoreszente Pigmente verarbeitet, die in blauem und ultraviolettem Licht zum Fluoreszieren angeregt werden können. Diese Fluoreszenz lässt Zahnrestaurationen in Sonnenlicht und künstlichem UV-Licht natürlich erscheinen, da auch natürliche Zähne eine Eigenfluoreszenz in diese Spektralbereich aufweisen. Die Fluoreszent bietet einfache Möglichkeit, Plaque, Zahnstein und Karies, aber auch Zahnrestaurationsmaterialien sichtbar zu machen.

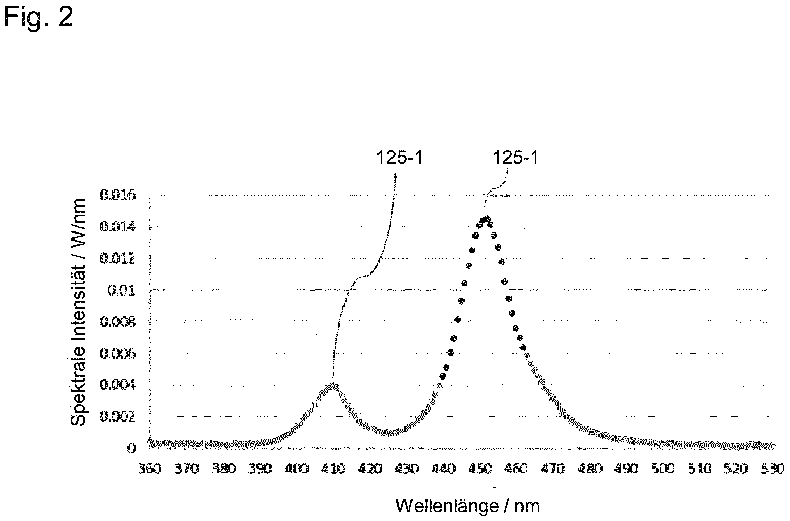

Fig. 2 zeigt ein Spektrum einer Lichtquelle 119 des Dentalhandgerätes 100. Das Spektrum der Lichtquelle 119 weist ein erstes Maximum 125-1 bei einer Wellenlänge von 410 nm und ein zweites Maximum 125-2 bei einer Wellenlänge von 450 nm auf.

Um eine Überstrahlung der Fluoreszenz auszuschließen, ist es vorteilhaft, lediglich mit kurzwelligem Licht anzuregen und längerwelliges Licht (450 nm) zu blockieren. Um beispielsweise eine Fluoreszenz sichtbar zu machen, wird das zweite Maximum 125--2 mittels eines optischen Filters 123 gesperrt. Das Dentalhandgerät 100 emittiert in diesem Fall nur Licht des ersten Maximums 125-1 im Wellenlängenbereich zwischen 380 nm und 415 nm.

Im Idealfall emittiert die Fluoreszenzanregungsvorrichtung 105 bei einem Anregungsmaximum von 405 nm (blau) und möglichst geringer Bandbreite. Das Zahnrestaurationsmaterial 103 fluoresziert beispielsweise bei einer Wellenlänge von 535 nm (grün) und Zahnsubstanz 107 mit Plaque, Zahnstein oder Karies fluoresziert bei einer Wellenlänge 610 nm (rot).

Das Dentalhandgerät 100 kann aber auch zwei unterschiedliche Lichtquellen 119 aufweisen, die jeweils in einem anderen Wellenlängenbereich strahlen. In diesem Fall kann eine Fluoreszenz sichtbar gemacht werden, wenn nur die eine Lichtquelle zum Anregen der Fluoreszenz eingeschaltet ist und die andere Lichtquelle ausgeschaltet ist. Die andere Lichtquelle dient zum Aushärten des Zahnrestaurationsmaterials 103.

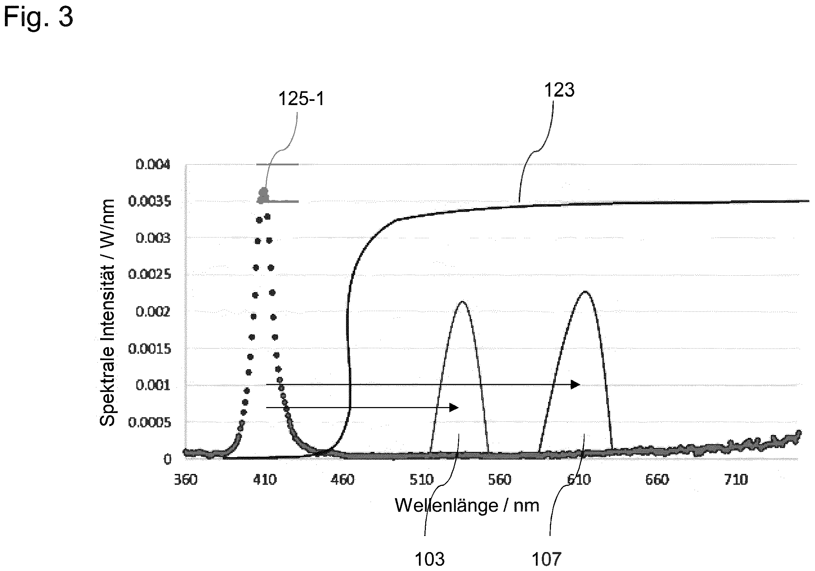

Fig. 3 zeigt ein weiteres Spektrum einer Lichtquelle des Dentalhandgerätes 100. Durch das Licht des ersten Maximums 125-1 im Wellenlängenbereich zwischen 380 nm und 415 nm wird die Fluoreszenz von Zahnsubstanz 107 im grünen Wellenlängenbereich (510 nm bis 550 nm) oder roten Wellenlängenbereich (580 nm bis 640 nm) angeregt. Da das optische Filter 123 eine Emission in Wellenlängenbereich größer als 460 nm sperrt, kann die Fluoreszenz gut erkannt werden. Das optische Filter 123 ist beispielsweise ein Hochpassfilter.

Zusätzlich kann ein Hochpassfilter in Form einer Brille oder eines Schildes zwischen einem Benutzer und dem Patienten genutzt werden, um eine Wellenlänge zu filtern und nur Fluoreszenzstrahlung mit längerer Wellenlänge durchzulassen.

Das Dentalhandgerät 100 ermöglicht die Integration der blaulichtinduzierten Fluoreszenzdiagnosemethode in einer dentalen Polymerisationslampe. Das Dentalhandgerät 100 vereint daher verschiedene Diagnosemöglichkeiten (Fluoreszenz von Zahnrestaurationsmaterialien und bakterieller Metaboliten) und Behandlung (Aushärtung von Zahnrestaurationsmaterial) in einem einzigen Gerät, um den Zahnarzt im Behandlungsablauf zu unterstützen. Das Dentalhandgerät 100 kann zudem verwendet werden, um übriggebliebenen Zahnstein und Plaque vor und nach der zahnhygienischen Behandlung zu erkennen.

Das Dentalhandgerät 100 erleichtert einen Workflow des Zahnarztes und ein Desinfizieren (Vermeidung der Cross-Kontamination). Zusätzliche Einsatzgebiete können in der Zahnreinigung oder Karies-Diagnostik liegen. Das Dentalhandgerät 100 erleichtert die Diagnose-Möglichkeit (Karies, alte Füllung) und ermöglicht eine Behandlung (Polymerisationslampe) in einem Gerät. Es erlaubt bei einer Füllungslegung alle Schritte mit demselben Gerät abzudecken (Kariesentfernung, Entfernung alter Füllungen, Aushärtung neuer Füllungen).

Die rote Fluoreszenz von bakteriengenerierten, zahnärztlichen Indikationen, wie Plaque, Zahnstein oder Karies, kann mit dem Dentalhandgerät 100 effizient dargestellt werden. Das Dentalhandgerät 100 bietet somit eine schnelle unkomplizierte Lösung in einem Gerät, das von einem Zahnarzt bereits verwendet wird.

### BEZUGSZEICHENLISTE

- 100: Dentalhandgerät
- 101: Aushärtevorrichtung
- 103: Zahnrestaurationsmaterial
- 105: Fluoreszenzanregungsvorrichtung
- 107: Zahnsubstanz
- 109: elektronisches Steuergerät
- 111: Zahn
- 113: Lichtleiter
- 115: elektronische Kamera
- 117: elektronische Datenschnittstelle
- 119: Lichtquelle
- 121: externes Gerät
- 123: optisches Filter
- 125: Maximum
- 127: Spiegel/Mikrospiegel

## Patentansprüche

1. Dentalhandgerät (100), mit:
einer Aushärtevorrichtung (101) zum Aushärten eines Zahnrestaurationsmaterials (103) mit Licht in einem ersten Wellenlängenbereich;
einer Fluoreszenzanregungsvorrichtung (105) zum Anregen einer Fluoreszenz von Zahnsubstanz (107) mit Licht in einem zweiten Wellenlängenbereich; und
einem elektronischen Steuergerät (109) zum Steuern der Aushärtevorrichtung (101) und der
Fluoreszenzanregungsvorrichtung (105);
**dadurch gekennzeichnet dass**, eine Intensität des Lichtes der Aushärtevorrichtung (101) und/oder des Lichtes der Fluoreszenzanregungsvorrichtung (105) einstellbar ist.

2. Dentalhandgerät (100) nach Anspruch 1, wobei das elektronische Steuergerät (109) ausgebildet ist, die Aushärtevorrichtung (101) und die Fluoreszenzanregungsvorrichtung (105) jeweils unabhängig voneinander zu aktivieren.

3. Dentalhandgerät (100) nach Anspruch 1 oder 2, wobei das elektronische Steuergerät (109) ausgebildet ist, die Aushärtevorrichtung (101) und die Fluoreszenzanregungsvorrichtung (105) in einem vorgegebenen zeitlichen Abstand zu aktivieren.

4. Dentalhandgerät (100) nach einem der vorangehenden Ansprüche, wobei das Dentalhandgerät (100) eine elektronische Kamera (115) zum Aufnehmen von Fluoreszenzlicht umfasst.

5. Dentalhandgerät (100) nach Anspruch 4, wobei das Dentalhandgerät (100) eine elektronische Datenschnittstelle (117) zum Übertragen von Daten der elektronischen Kamera (115) an ein externes Gerät (121) umfasst.

6. Dentalhandgerät (100) nach einem der vorangehenden Ansprüche, wobei das Dentalhandgerät (100) eine Lichtquelle (119) umfasst, die ausgebildet ist, Licht in dem ersten Wellenlängenbereich und dem zweiten Wellenlängenbereich zu emittieren.

7. Dentalhandgerät (100) nach einem der vorangehenden Ansprüche, wobei das Dentalhandgerät (100) ein optisches Filter (123) zum Sperren des ersten Wellenlängenbereichs und des zweiten Wellenlängenbereich umfasst.

8. Dentalhandgerät (100) nach Anspruch 7, wobei ein Strahlengang durch das optische Filter (123) umlenkbar ist oder das Filter (123) in den Strahlengang bewegbar ist.

9. Dentalhandgerät (100) nach Anspruch 8, wobei das Dentalhandgerät (100) einen beweglichen Spiegel (127) oder einen elektronisch ansteuerbaren Mikrospiegel (127) zum Umlenken des Strahlengangs durch das optische Filter (123) umfasst.

10. Dentalhandgerät (100) nach einem der Ansprüche 7 bis 9, wobei das Filter (123) ein Kantenfilter oder ein Hochpassfilter umfasst.

11. Dentalhandgerät (100) nach einem der vorangehenden Ansprüche, wobei das Dentalhandgerät (100) eine erste Lichtquelle zum Emittieren von Licht in dem ersten Wellenlängenbereich und eine zweite Lichtquelle zum Emittieren von Licht in dem zweiten Wellenlängenbereich umfasst.

12. Dentalhandgerät (100) nach einem der vorangehenden Ansprüche, wobei eine Aktivierung der Aushärtevorrichtung (101) und/oder der Fluoreszenzanregungsvorrichtung (105) auswählbar ist.

13. Dentalhandgerät (100) nach einem der vorangehenden Ansprüche, wobei der erste Wellenlängenbereich zwischen 380 nm und 515 nm und der zweite Wellenlängenbereich zwischen 380 nm und 415 nm liegt.

## Claims

1. A dental hand-held device (100), comprising:
a curing device (101) for curing a dental restoration material (103) with light in a first wavelength range;
a fluorescence excitation device (105) for exciting fluorescence of tooth substance (107) with light in a second wavelength range; and
an electronic controller (109) for controlling the curing device (101) and the fluorescence excitation device (105);
**characterized in that** an intensity of the light of the curing device (101) and/or of the light of the fluorescence excitation device (105) is adjustable.

2. The dental hand-held device (100) according to claim 1, wherein the electronic controller (109) is configured to activate the curing device (101) and the fluorescence excitation device (105) independently of each other.

3. The dental hand-held device (100) according to claim 1 or 2, wherein the electronic controller (109) is configured to activate the curing device (101) and the fluorescence excitation device (105) at a predetermined time interval.

4. The dental hand-held device (100) according to any one of the preceding claims, wherein the dental hand-held device (100) comprises an electronic camera (115) for capturing fluorescent light.

5. The dental hand-held device (100) according to claim 4, wherein the dental hand-held device (100) comprises an electronic data interface (117) for transmitting data from the electronic camera (115) to an external device (121) .

6. The dental hand-held device (100) according to any one of the preceding claims, wherein the dental hand-held device (100) comprises a light source (119) configured to emit light in the first wavelength range and the second wavelength range.

7. The dental hand-held device (100) according to any one of the preceding claims, wherein the dental hand-held device (100) comprises an optical filter (123) for blocking the first wavelength range and the second wavelength range.

8. The dental hand-held device (100) according to claim 7, wherein a beam path through the optical filter (123) is deflectable or the filter (123) is movable into the beam path.

9. The dental hand-held device (100) according to claim 8, wherein the dental hand-held device (100) comprises a movable mirror (127) or an electronically controllable micromirror (127) for redirecting the beam path through the optical filter (123).

10. The dental hand-held device (100) according to any one of claims 7 to 9, wherein the filter (123) comprises an edge filter or a high pass filter.

11. The dental hand-held device (100) according to any one of the preceding claims, wherein the dental hand-held device (100) comprises a first light source for emitting light in the first wavelength range and a second light source for emitting light in the second wavelength range.

12. The dental hand-held device (100) according to any one of the preceding claims, wherein activation of the curing device (101) and/or the fluorescence excitation device (105) is selectable.

13. The dental hand-held device (100) according to any one of the preceding claims, wherein the first wavelength range is between 380 nm and 515 nm and the second wavelength range is between 380 nm and 415 nm.

## Revendications

1. Appareil dentaire manuel (100), comprenant :
un dispositif de durcissement (101) pour durcir un matériau de restauration dentaire (103) avec de la lumière dans une première gamme de longueurs d'onde ;
un dispositif d'excitation de fluorescence (105) pour exciter une fluorescence de la substance dentaire (107) avec de la lumière dans une deuxième gamme de longueurs d'onde ; et
un dispositif de commande électronique (109) pour commander le dispositif de durcissement (101) et le dispositif d'excitation de fluorescence (105) ;
**caractérisé en ce qu'**une intensité de la lumière du dispositif de durcissement (101) et/ou de la lumière du dispositif d'excitation de fluorescence (105) est réglable.

2. Appareil dentaire manuel (100) selon la revendication 1, où le dispositif de commande électronique (109) est conçu pour activer le dispositif de durcissement (101) et le dispositif d'excitation de fluorescence (105), chacun indépendamment l'un de l'autre.

3. Appareil dentaire manuel (100) selon la revendication 1 ou 2, où l'appareil de commande électronique (109) est conçu pour activer le dispositif de durcissement (101) et le dispositif d'excitation de fluorescence (105) à un intervalle de temps prédéterminé.

4. Appareil dentaire manuel (100) selon l'une des revendications précédentes, où l'appareil dentaire manuel (100) comprend une caméra électronique (115) pour capter la lumière fluorescente.

5. Appareil dentaire manuel (100) selon la revendication 4, où l'appareil dentaire manuel (100) comprend une interface électronique de données (117) pour transmettre des données de la caméra électronique (115) à un appareil externe (121) .

6. Appareil dentaire manuel (100) selon l'une des revendications précédentes, où l'appareil dentaire manuel (100) comprend une source de lumière (119) configurée pour émettre de la lumière dans la première gamme de longueurs d'onde et dans la deuxième gamme de longueurs d'onde.

7. Appareil dentaire manuel (100) selon l'une des revendications précédentes, où l'appareil dentaire manuel (100) comprend un filtre optique (123) pour bloquer la première gamme de longueurs d'onde et la deuxième gamme de longueurs d'onde.

8. Appareil dentaire manuel (100) selon la revendication 7, où un trajet de faisceau peut être dévié par le filtre optique (123) ou le filtre (123) peut être déplacé dans le trajet de faisceau.

9. Appareil dentaire manuel (100) selon la revendication 8, où l'appareil dentaire manuel (100) comprend un miroir mobile (127) ou un micro-miroir (127) à commande électronique pour dévier le trajet des rayons à travers le filtre optique (123)

10. Appareil dentaire manuel (100) selon l'une quelconque des revendications 7 à 9, où le filtre (123) comprend un filtre de bord ou un filtre passe-haut.

11. Appareil dentaire manuel (100) selon l'une des revendications précédentes, où l'appareil dentaire manuel (100) comprend une première source lumineuse pour émettre de la lumière dans la première gamme de longueurs d'onde et une deuxième source lumineuse pour émettre de la lumière dans la deuxième gamme de longueurs d'onde.

12. Appareil dentaire manuel (100) selon l'une des revendications précédentes, où une activation du dispositif de durcissement (101) et/ou du dispositif d'excitation de fluorescence (105) peut être sélectionnée.

13. Appareil dentaire manuel (100) selon l'une des revendications précédentes, où la première gamme de longueurs d'onde est comprise entre 380 nm et 515 nm et la deuxième gamme de longueurs d'onde est comprise entre 380 nm et 415 nm.
